(19) Europäisches Patentamt
European Patent Office
Office européen des brevets

(11) EP 3 919 464 A1

(12) **EUROPEAN PATENT APPLICATION**
published in accordance with Art. 153(4) EPC

(43) Date of publication:
**08.12.2021 Bulletin 2021/49**

(21) Application number: **20748715.8**

(22) Date of filing: **28.01.2020**

(51) Int Cl.:
***C04B 40/04*** *(2006.01)* ***B28B 11/24*** *(2006.01)*
***E04G 21/02*** *(2006.01)*

(86) International application number:
**PCT/JP2020/003038**

(87) International publication number:
**WO 2020/158756 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2019 JP 2019013534**
**02.10.2019 JP 2019182382**

(71) Applicant: **Nippon Shokubai Co., Ltd.**
**Osaka-shi, Osaka 541-0043 (JP)**

(72) Inventors:
- **KAWAI Hajime**
  **Suita-shi, Osaka 564-0034 (JP)**
- **INUBUSHI Ryosuke**
  **Suita-shi, Osaka 564-0034 (JP)**

(74) Representative: **Hoffmann Eitle**
**Patent- und Rechtsanwälte PartmbB**
**Arabellastraße 30**
**81925 München (DE)**

(54) **CURING AGENT, METHOD FOR PRODUCING CEMENT STRUCTURE WITH COATING FILM, SHRINKAGE REDUCTION METHOD AND DRYING SUPPRESSION METHOD FOR CEMENT MOLDED BODY, AND METHOD FOR SUPPRESSING PENETRATION OF DETERIORATION FACTOR INTO CEMENT STRUCTURE**

(57) Provided is a curing agent containing a diester compound represented by the following Formula (I):

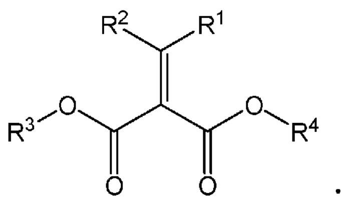

• • • (I)

wherein in Formula (I), $R^1$ and $R^2$ each independently represent a hydrogen atom or a monovalent hydrocarbon group having 1 to 15 carbon atoms, or $R^1$ and $R^2$ are bonded together to form a divalent hydrocarbon group having 3 to 15 carbon atoms; and $R^3$ and $R^4$ each independently represent a monovalent organic group having 1 to 30 carbon atoms, or $R^3$ and $R^4$ are bonded together to form a divalent organic group having 3 to 30 carbon atoms.

EP 3 919 464 A1

## Description

### Technical Field

The present disclosure relates to a curing agent, a method for producing a cement-based structure with a coating film, a shrinkage reduction method and a drying suppression method for a cement-based molded body, and a method for suppressing penetration of a deterioration factor into a cement-based structure.

### Background Art

Cement-based materials give hardened products having excellent strength, durability, and the like. From this point of view, cement-based materials are widely used as cement compositions such as cement paste, mortar, and concrete. Cement-based materials are materials that are indispensable for civil engineering or for constructing building structures.

A cement-based structure such as a building structure is obtained by casting and molding a cement composition into a predetermined shape to form a cement-based molded body, and then causing cement to react (curing) by means of the moisture inside the cement-based molded body. Here, during curing of the cement-based molded body, since moisture is dissipated from the surface of the cement-based molded body, drying shrinkage proceeds, defects such as cracks occur in the cement-based molded product, and there is a problem that strength and durability are lowered.

Therefore, as a method for suppressing the dissipation of moisture from the surface of a cement-based molded body during curing of the cement-based molded body, coating film curing in which curing is performed by forming a coating film on the surface of a cement-based molded body using a curing agent is known (see Patent Literature 1).

### Citation List

### Patent Document

Patent Literature 1: Japanese Laid-open Patent Publication No. 2010-18490

## Summary of Invention

### Technical Problem

However, with conventional curing agents, there is still room for improvements on preventing the dissipation of moisture, and there is a demand for the development of a curing agent with less dissipation of moisture.

The present disclosure was achieved in view of the above-described circumstances, and it is an object of the present disclosure to provide a curing agent that can effectively suppress dissipation of moisture from the surface of a cement-based molded body during curing. Furthermore, it is another object of the present disclosure to provide a method for producing a cement-based structure with a coating film by using such a curing agent, and a shrinkage reduction method and a drying suppression method for a cement-based molded body.

### Solution to Problem

The curing agent of the present disclosure contains a diester compound represented by the following Formula (I).

[Chemical Formula 1]

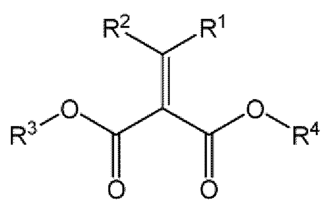

・・・(I)

wherein in Formula (I), $R^1$ and $R^2$ each independently represent a hydrogen atom or a monovalent hydrocarbon group having 1 to 15 carbon atoms, or $R^1$ and $R^2$ are bonded together to form a divalent hydrocarbon group having 3 to 15

carbon atoms; and $R^3$ and $R^4$ each independently represent a monovalent organic group having 1 to 30 carbon atoms, or $R^3$ and $R^4$ are bonded together to form a divalent organic group having 3 to 30 carbon atoms.

It is preferable that the above-described diester compound includes a first diester compound, and the first diester compound satisfies at least one of the following conditions (1) and (2).

(1) The glass transition temperature of a homopolymer is lower than 30°C.
(2) $R^3$ and $R^4$ each independently represent a monovalent linear or branched alkyl group having 3 to 30 carbon atoms.

It is preferable that the above-described diester compound further includes a second diester compound whose homopolymer has a glass transition temperature of 60°C or higher.

It is preferable that the glass transition temperature of a homopolymer of the above-described second diester compound is 100°C or higher.

It is preferable that the curing agent of the present disclosure further contains a polyfunctional methylenemalonic acid ester compound containing two or more of a structural unit represented by the following Formula (II) in the molecule and having the two or more structural units linked by a residue of a polyhydric alcohol.

[Chemical Formula 2]

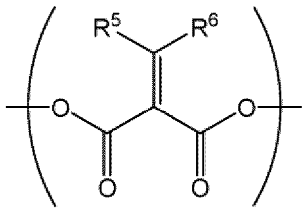

・・・(I I)

wherein in Formula (II), $R^5$ and $R^6$ each independently represent a hydrogen atom or a monovalent hydrocarbon group having 1 to 15 carbon atoms.

It is preferable that the method for producing a cement-based structure with a coating film of the present disclosure includes a step of coating at least a portion of the surface of a cement-based molded body with the above-described curing agent and then curing the cement-based molded body.

The shrinkage reduction method for the cement-based molded body of the present disclosure includes coating at least a portion of the surface of a cement-based molded body with the above-described curing agent, and thereby reducing shrinkage of the cement-based molded body during curing.

The drying suppression method for the cement-based molded body of the present disclosure includes coating at least a portion of the surface of a cement-based molded body with the above-described curing agent, and thereby suppressing drying of the cement-based molded body during curing.

Furthermore, the method for suppressing penetration of a deterioration factor into a cement-based structure of the present disclosure includes coating at least a portion of the surface of a cement-based structure with a surface protective agent containing a diester compound represented by the above-described Formula (I), and thereby suppressing penetration of a deterioration factor into the cement-based structure.

## Advantageous Effects of Invention

According to the present disclosure, a curing agent that can effectively suppress dissipation of moisture from the surface of a cement-based molded body during curing can be provided. Furthermore, according to the present disclosure, a method for producing a cement-based structure with a coating film by using such a curing agent, and a shrinkage reduction method and a drying suppression method for a cement-based molded body can be provided.

## Description of Embodiments

The curing agent of the present disclosure is a curing agent for a cement-based molded body and contains a diester compound represented by the following Formula (I). According to such a curing agent, dissipation of moisture from the surface of a cement-based molded body during curing can be effectively suppressed. Therefore, the curing agent of the present disclosure can reduce dry shrinkage of a cement-based molded body during curing and can effectively suppress the occurrence of cracks and the like caused by dry shrinkage. Furthermore, when curing is performed using the curing agent of the present disclosure, dissipation of moisture from the cement-based molded body during curing

can be effectively prevented, and a hydraulic reaction of cement can be carried out more smoothly, so that there is a tendency that the compressive strength of the resulting cement-based structure can be increased.

[Chemical Formula 3]

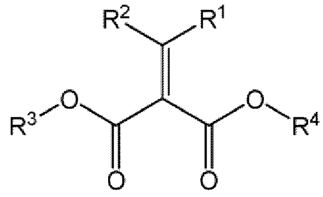

・・・(I)

wherein in Formula (I), $R^1$ and $R^2$ each independently represent a hydrogen atom or a monovalent hydrocarbon group having 1 to 15 carbon atoms, or $R^1$ and $R^2$ are bonded together to form a divalent hydrocarbon group having 3 to 15 carbon atoms; and $R^3$ and $R^4$ each independently represent a monovalent organic group having 1 to 30 carbon atoms, or $R^3$ and $R^4$ are bonded together to form a divalent organic group having 3 to 30 carbon atoms.

In Formula (I), the carbon number of the hydrocarbon group for $R^1$ and $R^2$ is preferably 1 to 10, and preferably 1 to 5. Specific examples of $R^1$ and $R^2$ include a methyl group, an ethyl group, an n-butyl group, an n-pentyl group (amyl group), an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an isopropyl group, a 2-methylbutyl group, an isoamyl group, a 3,3-dimethylbutyl group, a 2-ethylbutyl group, a 2-ethyl-2-methylpropyl group, an isoheptyl group, an isooctyl group, a 2-ethylhexyl group, a 2-propylpentyl group, a neononyl group, a 2-ethylheptyl group, a 2-propylhexyl group, a 2-butylpentyl group, an isodecyl group, a neodecyl group, a 2-ethyloctyl group, a 2-propylheptyl group, a 2-butylhexyl group, an isoundecyl group, a neoundecyl group, a 2-ethylnonyl group, a 2-propyloctyl group, a 2-butylheptyl group, a 2-pentylhexyl group, an isododecyl group, a neododecyl group, a 2-ethyldecyl group, a 2-propylnonyl group, a 2-butyloctyl group, a 2-pentylheptyl group, an isotridecyl group, a neotridecyl group, a 2-ethylundecyl group, a 2-propyldecyl group, a 2-butyloctyl group, a 2-pentyloctyl group, a 2-hexylheptyl group, an isotetradecyl group, a neotetradecyl group, a 2-ethyldodecyl group, a 2-propylundecyl group, a 2-butyldecyl group, a 2-pentylnonyl group, a 2-hexyloctyl group, an isopentadecyl group, a neopentadecyl group, a cyclohexylmethyl group, and a benzyl group.

In Formula (I), regarding $R^1$ and $R^2$, at least one of them may be a hydrogen atom, or both may be hydrogen atoms. Incidentally, it is preferable that both $R^1$ and $R^2$ are hydrogen atoms.

In a case where $R^1$ and $R^2$ are bonded together to form a divalent hydrocarbon group having 3 to 15 carbon atoms, the carbon number of the divalent hydrocarbon group is preferably 4 to 12, and more preferably 5 to 9. Specific examples of the divalent hydrocarbon group include a 1,3-propylene group, a 1,4-butylene group, a 1,5-pentylene group, a 1,6-hexylene group, and a 1,5-hexylene group.

In Formula (I), $R^3$ and $R^4$ are monovalent organic groups, and examples of such an organic group include a monovalent hydrocarbon group and a monovalent heteroatom-containing group. The monovalent hydrocarbon group and monovalent heteroatom-containing group may each have a substituent. Examples of the above-described substituent include an alkoxy group, a hydroxy group, a nitro group, an azido group, a cyano group, an acyl group, an acyloxy group, a carboxyl group, a heterocyclic group, an ester group, and a residue of another monomer (B), and these may be further substituted with substituents. The carbon numbers of $R^3$ and $R^4$ are each 1 to 30, preferably 1 to 20, more preferably 1 to 15, and even more preferably 1 to 10. In a case where each of $R^3$ and $R^4$ has one or two or more substituents, it is preferable that the carbon numbers including the substituents are each in the above-described range of carbon number. There are no limitations on the number of substituents for $R^3$ and $R^4$; however, the number of substituents is preferably 5 or less, more preferably 3 or less, and even more preferably 1 or 2, for each group. Examples of the monovalent heteroatom-containing group include a polyalkylene oxide group and a polyester group.

The hydrocarbon group may be either an aliphatic hydrocarbon group or an aromatic hydrocarbon group, and the aliphatic hydrocarbon group may be any of a linear aliphatic hydrocarbon group, a branched aliphatic hydrocarbon group, or an alicyclic hydrocarbon group. Furthermore, the aliphatic hydrocarbon group may be either a saturated aliphatic hydrocarbon group or an unsaturated aliphatic hydrocarbon group. Incidentally, the aromatic hydrocarbon group is a group having an aromatic ring and may have an aliphatic moiety. The alicyclic hydrocarbon group is a group having a cyclic aliphatic hydrocarbon moiety and may have a linear or branched aliphatic hydrocarbon moiety.

Examples of the linear saturated hydrocarbon group include a methyl group, an ethyl group, an n-propyl group, an n-butyl group, an n-pentyl group (amyl group), an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-eicosyl group, an n-heneicosyl group, and an n-docosyl group.

Examples of the branched saturated hydrocarbon group include an isopropyl group, a sec-butyl group, an isobutyl group, a tert-butyl group, a 1-methylbutyl group, a 1-ethylpropyl group, a 2-methylbutyl group, an isoamyl group, a 1,2-dimethylpropyl group, a 1,1-dimethylpropyl group, a tert-amyl group, a 1,3-dimethylbutyl group, a 3,3-dimethylbutyl group, a 1-methylpentyl group, a 1-methylbutyl group, a 1-ethylbutyl group, a 2-ethylbutyl group, a 2-ethyl-2-methylpropyl group, a sec-heptyl group, a tert-heptyl group, an isoheptyl group, a sec-octyl group, a tert-octyl group, an isooctyl group, a 1-ethylhexyl group, a 1-propylpentyl group, a 2-ethylhexyl group, a 2-propylpentyl group, a sec-nonyl group, a tert-nonyl group, a neononyl group, a 1-ethylheptyl group, a 1-propylhexyl group, a 1-butylpentyl group, a 2-ethylheptyl group, a 2-propylhexyl group, a 2-butylpentyl group, an isodecyl group, a sec-decyl group, a tert-decyl group, a neodecyl group, a 1-ethyloctyl group, a 1-propylheptyl group, a 1-butylhexyl group, a 2-ethyloctyl group, a 2-propylheptyl group, a 2-butylhexyl group, an isoundecyl group, a sec-undecyl group, a tert-undecyl group, a neoundecyl group, a 1-ethylnonyl group, a 1-propyloctyl group, a 1-butylheptyl group, a 1-pentylhexyl group, a 2-ethylnonyl group, a 2-propyloctyl group, a 2-butylheptyl group, a 2-pentylhexyl group, an isododecyl group, a sec-dodecyl group, a tert-dodecyl group, a neododecyl group, a 1-ethyldecyl group, a 1-propylnonyl group, a 1-butyloctyl group, a 1-pentylheptyl group, a 2-ethyldecyl group, a 2-propylnonyl group, a 2-butyloctyl group, a 2-pentylheptyl group, an isotridecyl group, a sec-tridecyl group, a tert-tridecyl group, a neotridecyl group, a 1-ethylundecyl group, a 1-propyldecyl group, a 1-butylnonyl group, a 1-pentyloctyl group, a 1-hexylheptyl group, a 2-ethylundecyl group, a 2-propyldecyl group, a 2-butyloctyl group, a 2-pentyloctyl group, a 2-hexylheptyl group, an isotetradecyl group, a sec-tetradecyl group, a tert-tetradecyl group, a neotetradecyl group, a 1-ethyldodecyl group, a 1-propylundecyl group, a 1-butyldecyl group, a 1-pentylnonyl group, a 1-hexyloctyl group, a 2-ethyldodecyl group, a 2-propylundecyl group, a 2-butyldecyl group, a 2-pentylnonyl group, a 2-hexyloctyl group, an isopentadecyl group, a sec-pentadecyl group, a tert-pentadecyl group, a neopentadecyl group, an isohexadecyl group, a sec-hexadecyl group, a tert-hexadecyl group, a neohexadecyl group, an isoheptadecyl group, a sec-heptadecyl group, a tert-heptadecyl group, a neoheptadecyl group, an isooctadecyl (isostearyl) group, a sec-octadecyl group, a tert-octadecyl group, a neooctadecyl group, an isononadecyl group, a sec-nonadecyl group, a tert-nonadecyl group, a neononadecyl group, an isoeicosyl group, a sec-eicosyl group, a tert-eicosyl group, a neoeicosyl group, an isoheneicosyl group, a sec-heneicosyl group, a tert-heneicosyl group, a neoheneicosyl group, an isodocosyl group, a sec-docosyl group, a tert-docosyl group, a neodocosyl group, an isotricosyl group, a sec-tricosyl group, a tert-tricosyl group, a neotricosyl group, an isotetracosyl group, a sec-tetracosyl group, a tert-tetracosyl group, a neotetracosyl group, an isopentacosyl group, a sec-pentacosyl group, a tert-pentacosyl group, a neopentacosyl group, an isohexacosyl group, a sec-hexacosyl group, a tert-hexacosyl group, a neohexacosyl group, an isoheptacosyl group, a sec-heptacosyl group, a tert-heptacosyl group, a neoheptacosyl group, an isooctacosyl group, a sec-octacosyl group, a tert-octacosyl group, a neooctacosyl group, an isononacosyl group, a sec-nonacosyl group, a tert-nonacosyl group, a neononacosyl group, an isotriacontyl group, a sec-triacontyl group, and a tert-triacontyl group.

Examples of the alicyclic hydrocarbon group include a cyclopentyl group, a cyclohexyl group, a cycloheptyl group, a cyclooctyl group, a methylcyclohexyl group, a cyclohexylmethyl group, an adamantyl group, and a norbornyl group.

The unsaturated hydrocarbon group may be a linear alkenyl group or a branched alkenyl group, and specific examples of the linear alkenyl group include a vinyl group, an allyl group, a 1-butenyl group, a 2-butenyl group, a pentenyl group, a hexenyl group, a heptenyl group, an octenyl group, a nonenyl group, a decenyl group, a dodecenyl group, an octadecenyl group, and an eicosenyl group.

Examples of the branched alkenyl group include an isopropenyl group, an isobutenyl group, an isopentenyl group, an isohexenyl group, an isoheptenyl group, an isooctenyl group, an isononenyl group, an isodecenyl group, an isododecenyl group, an isooctadecenyl group, and an isoeicosenyl group.

Examples of the aromatic hydrocarbon group include a phenyl group; a naphthyl group; an aralkyl group such as a benzyl group, a 1-phenylethyl group, a 2-phenylethyl group, a 3-phenylpropyl group, or a 4-phenylbutyl group; a styryl group (Ph-CH=C- group); a cinnamyl group ($Ph-CH=CHCH_2-$ group); a 1-benzocyclobutenyl group; a 1,2,3,4-tetrahydronaphthyl group, and a distyrenated phenyl group.

In a case where $R^3$ and $R^4$ are bonded together to form a divalent organic group having 3 to 30 carbon atoms, the carbon number of the divalent organic group is preferably 3 to 10, and more preferably 3 to 6. Furthermore, the divalent organic group may be a divalent hydrocarbon group, and specific examples of the divalent hydrocarbon group include a 2,2-propylene group, a 1,3-propylene group, a 1,4-butylene group, a 1,5-pentylene group, a 1,6-hexylene group, and a 1,5-hexylene group. This divalent organic group may be a group obtained by substituting one or more hydrogen atoms of a divalent hydrocarbon group having 3 to 15 carbon atoms with substituents. Examples of the substituent include the substituents mentioned as examples in a case where the above-mentioned $R^3$ and $R^4$ are monovalent organic groups. It is preferable that the divalent organic group has 1 to 5 or fewer substituents, and it is more preferable that the divalent organic group has 1 to 3 substituents.

The divalent organic group may be a divalent heteroatom-containing group, and examples of the divalent heteroatom-containing group include a polyalkylene oxide group and a polyester group.

Examples of the diester compound represented by Formula (I) include methyl propyl methylenemalonate, di-n-hexyl methylenemalonate, dicyclohexyl methylenemalonate, diisopropyl methylenemalonate, butyl methyl methylenemalonate, ethoxyethyl ethyl methylenemalonate, methoxyethyl methyl methylenemalonate, hexyl ethyl methylenemalonate, di-n-pentyl methylenemalonate, ethyl pentyl methylenemalonate, methyl pentyl methylenemalonate, ethyl ethylmethoxyl methylenemalonate, ethoxyethyl methyl methylenemalonate, butyl ethyl methylenemalonate, di-n-butyl methylenemalonate, diethyl methylenemalonate (DEMM), diethoxyethyl methylenemalonate, dimethyl methylenemalonate, di-n-propyl methylenemalonate, ethyl hexyl methylenemalonate, fenchyl methyl methylenemalonate, menthyl methyl methylenemalonate, 2-phenylpropyl ethyl methylenemalonate, 3-phenyl propyl methylenemalonate, dimethoxyethyl methylenemalonate, di-n-heptyl methylenemalonate, di-n-octyl methylenemalonate, di-n-nonyl methylenemalonate, and di-n-decyl methylenemalonate. Among these, di-n-hexyl methylenemalonate and dicyclohexyl methylenemalonate are preferred.

The content of the diester compound represented by Formula (I) in the curing agent is not particularly limited; however, the content may be 50% by mass or more, 80% by mass or more, 90% by mass or more, or 95% by mass or more, with respect to the total mass of the curing agent.

The diester compound represented by Formula (I) is preferably a first diester compound that satisfies at least one of the following conditions (1) and (2). As the curing agent includes the first diester compound, the hardening rate at the time of applying the curing agent on a cement-based molded body tends to be higher. Meanwhile, in the following description, with regard to the diester compound of Formula (I), the glass transition temperature of a homopolymer is simply referred to as Tg.

(1) The glass transition temperature of a homopolymer is lower than 30°C.
(2) $R^3$ and $R^4$ each independently represent a monovalent linear or branched alkyl group having 3 to 30 carbon atoms.

With regard to the condition (1), the Tg of the first diester compound is preferably 25°C or lower, more preferably 20°C or lower, even more preferably 10°C or lower, and particularly preferably 0°C or lower. Incidentally, the glass transition point of the homopolymer can be measured by, for example, differential scanning calorimetry (DSC), differential thermal analysis (DTA), thermomechanical analysis (TMA), or the like.

With regard to the condition (2), the carbon number of the alkyl group of $R^3$ and $R^4$ is preferably 3 to 12, preferably 4 to 10, and more preferably 5 to 10.

Examples of the first diester compound include di-n-butyl methylenemalonate, di-n-pentyl methylenemalonate, di-n-hexyl methylenemalonate, di-n-heptyl methylenemalonate, di-n-octyl methylenemalonate, di-n-nonyl methylenemalonate, and di-n-decyl methylenemalonate.

The content of the first diester compound in the curing agent is not particularly limited; however, the content is preferably 30% by mass or more, and more preferably 40% by mass or more, with respect to the total amount of the curing agent. Furthermore, the content of the first diester compound in the curing agent may be 95% by mass or less or may be 80% by mass or less, with respect to the total amount of the curing agent.

Regarding the diester compound represented by Formula (I), a second diester compound whose homopolymer has a glass transition temperature of 60°C or higher may also be used in combination with the first diester compound. As the first diester compound and the second diester compound are used in combination, tackiness of the coating film is decreased, and handling is made easier.

The Tg of the second diester compound is preferably 80°C or higher, and more preferably 100°C or higher. Furthermore, the content of the second diester compound is preferably 30% to 70% by mass, and more preferably 40% to 60% by mass, with respect to the total amount of the curing agent.

The second diester compound may be a compound having an alicyclic hydrocarbon group or an aromatic hydrocarbon group as $R^3$ and $R^4$ of Formula (I), and specific examples include fenchyl ethyl methylenemalonate, menthyl ethyl methylenemalonate, phenylpropyl methyl methylenemalonate, phenylpropyl ethyl methylenemalonate, and dicyclohexyl methylenemalonate.

The curing agent of the present disclosure may include components other than the diester compound represented by Formula (I). Examples of such components include an anionic polymerization inhibitor, a radical polymerization inhibitor, and an oxidation inhibitor. Furthermore, in addition to those, examples of the above-described components include a polyfunctional methylenemalonic acid ester compound which contains two or more units of a structural unit represented by the following Formula (II) in the molecule and in which two or more units of the structural unit are linked by a residue of a polyhydric alcohol. This residue of a polyhydric alcohol is an n-valent organic group obtained by eliminating n units of hydroxy groups from a polyhydric alcohol (number of residues of the diester compound of Formula (I) linked by this polyhydric alcohol).

[Chemical Formula 4]

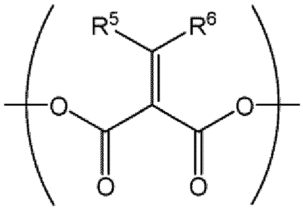

・・・(I I)

wherein in Formula (II), $R^5$ and $R^6$ each independently represent a hydrogen atom or a monovalent hydrocarbon group having 1 to 15 carbon atoms.

The above-described polyfunctional methylenemalonic acid ester compound may be a reaction product obtained by reacting a diester compound represented by Formula (I) with a polyhydric alcohol under the conditions in which a transesterification reaction occurs between the two compounds. Therefore, regarding specific examples of $R^5$ and $R^6$, those mentioned as examples of $R^1$ and $R^2$ in the above-mentioned Formula (I) may be mentioned.

Examples of the polyhydric alcohol include a dihydric alcohol and a trihydric or higher-hydric alcohol. Examples of the dihydric alcohol include an alkylene glycol and a polyalkylene glycol, and it is preferable that each of them has 2 to 30 carbon atoms, more preferably 2 to 20 carbon atoms, even more preferably 2 to 15 carbon atoms, and particularly preferably 2 to 10 carbon atoms. The carbon number of the trihydric or higher-hydric alcohol is preferably 3 to 30, more preferably 3 to 20, even more preferably 3 to 15, and particularly preferably 3 to 10. Examples of the polyhydric alcohol include dihydric alcohols such as ethylene glycol, butylene glycol, polyethylene glycol, and polypropylene glycol; and trihydric or higher-hydric alcohols such as glycerin, polyglycerin, a compound obtained by adding an alkylene glycol to glycerin, erythritol, xylitol, sorbitol, trimethylolpropane, pentaerythritol, and dipentaerythritol.

Regarding the above-described polyfunctional methylenemalonic acid ester compound, for example, a compound having a structure of the following Formula (IIA) or (IIB) may be mentioned.

[Chemical Formula 5]

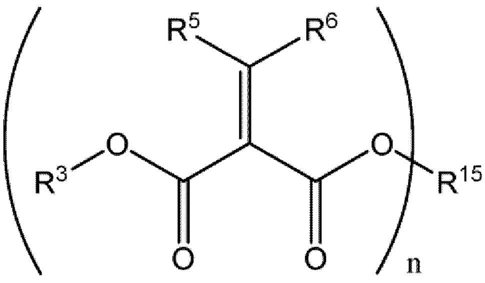

・・・(I I A)

wherein in Formula (IIA), $R^5$ and $R^6$ each independently represent a hydrogen atom or a hydrocarbon group having 1 to 15 carbon atoms, or $R^5$ and $R^6$ are bonded together to form a divalent hydrocarbon group having 3 to 15 carbon atoms; $R^3$ each independently represents a monovalent organic group having 1 to 30 carbon atoms; n represents the number of structural units within the parentheses included in the compound of Formula (IIA) and represents a number of 2 or larger; $R^{15}$ represents an n-valent organic group; a plurality of $R^5$'$_S$ may be identical with or different from each other; a plurality of $R^6$'s may be identical with or different from each other; and a plurality of $R^3$'s may be identical with or different from each other.

[Chemical Formula 6]

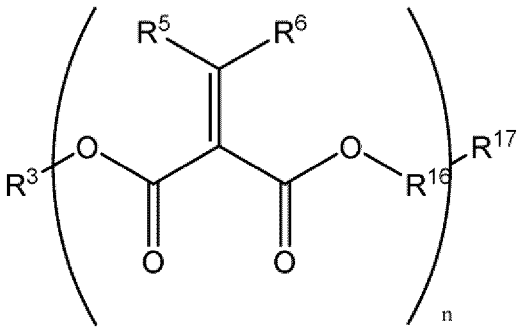

・・・(IIB)

wherein in Formula (IIB), $R^5$ and $R^6$ each independently represent a hydrogen atom or a hydrocarbon group having 1 to 15 carbon atoms, or $R^5$ and $R^6$ are bonded together to form a divalent hydrocarbon group having 3 to 15 carbon atoms; $R^3$ represents a monovalent organic group having 1 to 30 carbon atoms; n represents the number of structural units within the parentheses included in the compound of Formula (IIA) and represents a number of 2 or larger; $R^{16}$ represents a divalent organic group; $R^{17}$ represents a monovalent organic group and is preferably a hydroxy group or a group represented by the following Formula (III); a plurality of $R^{5}$'$_S$ may be identical with or different from each other; a plurality of $R^6$'s may be identical with or different from each other; a plurality of $R^{16}$'s may be identical with or different from each other; and $R^{17}$ may be a residue obtained by eliminating $R^3$ from a compound represented by Formula (I).

[Chemical Formula 7]

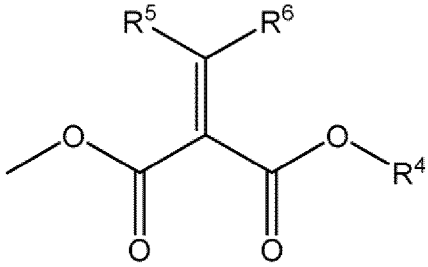

・・・(III)

wherein in Formula (III), $R^5$ and $R^6$ each independently represent a hydrogen atom or a hydrocarbon group having 1 to 15 carbon atoms, or $R^5$ and $R^6$ are bonded together to form a divalent hydrocarbon group having 3 to 15 carbon atoms; and $R^4$ each independently represent a monovalent organic group having 1 to 30 carbon atoms.

In Formula (IIA), $R^{15}$ represents an n-valent organic group and is a divalent or higher-valent organic group; however, $R^{15}$ is, for example, a residue obtained by eliminating two or more hydroxy groups from a polyol. Examples of the polyol include glycerin, polyglycerin, a compound obtained by adding an alkylene glycol to glycerin, erythritol, xylitol, sorbitol, trimethylolpropane, pentaerythritol, and dipentaerythritol. The upper limit of n is not particularly limited; however, the upper limit is preferably 100 or less, more preferably 12 or less, and even more preferably 6 or less. The carbon number of $R^{15}$ is each preferably 1 to 30, more preferably 1 to 20, even more preferably 1 to 15, and particularly preferably 1 to 10.

In Formula (IIB), $R^{16}$ represents a divalent organic group and is not particularly limited; however, it is preferable that $R^{16}$ is an organic group having 1 to 30 carbon atoms. Preferred examples include a residue obtained by eliminating two hydroxy groups from a diol, and a residue obtained by eliminating two hydroxy groups from a polyalkylene glycol. Examples of the above-described diol or polyalkylene glycol include ethylene glycol, butylene glycol, polyethylene glycol, and polypropylene glycol. The carbon number of $R^{16}$ is each preferably 1 to 30, more preferably 1 to 20, even more preferably 1 to 15, and particularly preferably 1 to 10.

In each of Formulae (IIA), (IIB), and (III), at least one of $R^5$ and $R^6$ may be a hydrogen atom, or both may be hydrogen atoms. Incidentally, in each of Formulae (IIA), (IIB), and (III), it is preferable that both $R^5$ and $R^6$ are hydrogen atoms.

The content of the above-described polyfunctional methylenemalonic acid ester compound is preferably 30% to 70% by mass, and more preferably 40% to 60% by mass, with respect to the total amount of the curing agent.

Incidentally, in a case where the first and second diester compounds and the polyfunctional methylenemalonic acid ester compound are used in combination, the content of the polyfunctional methylenemalonic acid ester compound is preferably 1% to 20% by mass, and more preferably 2% to 15% by mass, with respect to the total amount of the curing agent. In this case, the contents of the first and second diester compounds are each preferably 30% to 70% by mass, and more preferably 40% to 60% by mass, with respect to the total amount of the curing agent.

As the anionic polymerization inhibitor, an acid having an acid dissociation constant in water of 2 or less is preferred, and specific examples include sulfuric acid; sulfonic acids such as methanesulfonic acid and p-toluenesulfonic acid; sulfurous acid, phosphoric acid, and trifluoroacetic acid. In a case where the curing agent includes an anionic polymerization inhibitor, the content thereof may be appropriately adjusted according to the acidity; however, from the viewpoint of achieving a balance between storage stability and reactivity, the content is preferably 0.1 to 2000 ppm by mass, more preferably 1 to 1000 ppm by mass, and even more preferably 3 to 500 ppm by mass, with respect to the total amount of the diester compound of Formula (I) (in the case of including a polyfunctional methylenemalonic acid ester compound, the total amount of the diester compound of Formula (I) and the polyfunctional methylenemalonic acid ester compound). As the radical polymerization inhibitor and the oxidation inhibitor, hindered phenols, sulfur-based oxidation inhibitors, and phosphorus-based oxidation inhibitors are preferred from the viewpoint of suppressing coloration, and specific examples include hindered phenols such as 2,6-dit-butyl-4-methylphenol, 3-(3,5-di-t-butyl-4-hydroxyphenyl)propionic acid stearate, 2,2'-methylenebis(4-methyl-6-t-butylphenol), tetrakis(methylene-3-(3,5-di-t-butyl-4-hydroxyphenyl)propionate)methane, 4,4'-thiobis(3-methyl-6-t-butylphenol), and 2,5-di-t-butylhydroquinone; sulfur-based oxidation inhibitors such as dilauryl thiodipropionate and distearyl thiodipropionate; and phosphorus-based oxidation inhibitors such as triphenyl phosphite, tris(nonylphenyl) phosphite, distearylpentaerythritol diphosphite, and tetra(tridecyl)-1,1,3-tris(2-methyl-5-t-butyl-4-hydroxyphenyl)butane diphosphite. In a case where the curing agent includes a radical polymerization inhibitor or an oxidation inhibitor, from the viewpoint of achieving a balance between storage stability and reactivity, the content thereof is preferably 50 to 5000 ppm by mass, more preferably 100 to 3000 ppm by mass, and even more preferably 200 to 2000 ppm by mass, with respect to the total amount of the diester compound of Formula (I) (in the case of including a polyfunctional methylenemalonic acid ester compound, the total amount of the diester compound of Formula (I) and the polyfunctional methylenemalonic acid ester compound).

It is preferable that the curing agent according to the present embodiment substantially does not include an anionic polymerization initiator. Regarding the anionic polymerization initiator, for example, at least one anionic polymerization initiator selected from an organic base, an inorganic base, a metal oxide, a metal salt, and an ionic liquid may be mentioned. The content of such an anionic polymerization initiator is more preferably 0.02% by mass or less based on the total amount of the curing agent, and it is even more preferable that the curing agent does not include an anionic polymerization initiator. Furthermore, it is preferable that the curing agent according to the present embodiment is a one-liquid type curing agent from the viewpoint of workability. The term "one-liquid type" refers to an agent that is used without mixing with other components such as an anionic polymerization initiator at the time of use. Incidentally, in the case of a one-liquid type curing agent, the curing agent may include no anionic polymerization initiator at all; however, it is also acceptable that a very small amount of an anionic polymerization initiator is included if the anionic polymerization initiator has almost no effect on the pot life of the curing agent.

Examples of the organic base as the anionic polymerization initiator include acetates such as sodium acetate, potassium acetate, zinc acetate, copper acetate, and piperidine acetic acid; chloroacetates such as sodium chloroacetate, potassium chloroacetate, and copper chloroacetate; propionates such as sodium propionate; benzoates such as sodium benzoate; ammonium salts such as tetrabutylammonium fluoride, tetrabutylammonium chloride, and tetrabutylammonium hydroxide; amine compounds such as 2-dimethylaminoethylphenol, N,N,N',N'-tetrakis(2-hydroxyethyl)ethylenediamine, piperidine, piperazine, N-methylpiperazine, N,N-dimethylpiperazine, morpholine, 4-methylmorpholine, 2,2'-dimorpholinodiethyl ether, pyridine, imidazole, 1-methylimidazole, tetramethylguanidine (TMG), triethylamine, tris(dimethylaminoethyl)phenol, 2-dimethylaminoethylphenol, ethylhexylamine, N-octylamine, tridecylamine, 3,3'-dimethyl-4,4'-diaminodicyclohexylmethane, 4,4'-diaminodicyclohexylmethane, isophoronediamine, neopentanediamine (2,2-dimethylpropane-1,3-diamine), octamethylenediamine, dibutylethanolamine, 4,4'-diaminodiphenylmethane, benzylamine, N,N-dimethylbenzylamine, N,N-diethylbenzylamine, N,N-dibutylbenzylamine, N,N-dihexylbenzylamine, polyetheramine, di(2-ethylhexyl)amine, dibutylamine, dicyclohexylamine, ditridecylamine, 4,9-dioxadodecane-1,12-diamine, di(2-methoxyethyl)amine, dimethylethylamine, dimethylpropylamine, N,N-dimethylisopropylamine, N-ethyldiisopropylamine, N,N-dimethylcyclohexylamine, trimethylamine, triethylamine, tripropylamine, tributylamine, tris(2-ethylhexyl)amine, 2-(diisopropylamino)ethylamine, tetramethyl-1,6-hexanediamine, S-triazine, pentamethyldiethylenetriamine, bis(2-dimethylaminoethyl) ether, N,N-dimethylcyclohexylamine, bis(2-dimethylaminoethyl) ether, pentamethyldiethylenetriamine, trimethylaminoethylethanolamine, tetramethyl-1,6-hexanediamine, tris(dimethylaminomethyl)phenol, 2-methylaminomethylphenol, 3-(cyclohexylamino)propylamine, diethylenetriamine, dipropylenetriamine, 3-(2-aminoethylamino)propylamine, N,N'-bis(3-aminopropyl)ethylenediamine, 3-(diethylamino)propylamine, N,N-bis(3-aminopropyl)methylamine, butyldiethanolamine, triisopropanolamine, diethylethanolamine, methyldiethanolamine, methyldiisopropanolamine, N,N-dimethylethanolamine S, N,N-dimethylisopropanolamine, dimethylethanolamine, N,N,N',N'-tetrakis(2-hydroxyethyl)ethylenediamine, dimethylaminoethoxyethanol, 1,4-diazabicyclo[2.2.2]octane (DABCO), 1,1'-iminobis-2-propanol (DIPA), 1,2-cyclohexanamine, 1,3-cyclohexanedimethanamine, 2-methylpentamethylenediamine, 3,3-iminodipropylamine, triacetonediamine (TAD), 2-(dimethylamino)ethanol (DEME), 2-piperazin-1-ylethylamine, N,N,N',N'-tetrakis(2-hydroxypropyl)ethylenediamine, 2-[2-(dimethylamino)ethoxy]ethanol, 1-[bis[3-(dimethylamino)propyl]amino]-2-propanol, N,N,N',N",N"-pentamethyldiethylenetriamine, N,N,N',N'-tetraethyl-1,3-propanediamine, N,N,N',N'-tetramethyl-1,4-butanediamine, N,N,N',N'-tetramethyl-1,6-hexanediamine, 1,4,8,11-tetramethyl-1,4,8,11-tetraazacyclotetradecane, 1,3,5-trimethylhexahydro-1,3,5-triazine, 1,8-diazabicycloundec-7-ene (DBU), 1,5-diazabicyclo[4.3.0]non-5-ene (DBN), 1,1,3,3-tetramethylguanidine, 1,5,7-triazabicyclo[4.4.0]dec-5-ene, and 7-methyl-1,5,7-triazabicyclo[4.4.0]dec-5-ene; and amides.

Examples of the inorganic base as the anionic polymerization initiator include silicates such as sodium silicate, potassium silicate, and magnesium silicate; and hydroxides such as sodium hydroxide, potassium hydroxide, and calcium hydroxide.

Examples of the metal oxide as the anionic polymerization initiator include sodium oxide, potassium oxide, and calcium oxide.

Examples of the metal salt as the anionic polymerization initiator include lithium chloride.

An ionic liquid as the anionic polymerization initiator refers to a salt that usually contains a cation and an anion and has a melting point of lower than 100°C at 1 atmosphere, and for example, a salt that is liquid at room temperature (for example, 23°C) may be mentioned. Examples of the cation for the ionic liquid include a cation having a nitrogen-containing heterocyclic structure, imidazolium cation, pyrazolium cation, pyrrolidinium cation, pyridinium cation, pyrazinium cation, pyrimidinium cation, and derivatives thereof. Examples of a cation other than those include a tetraalkylphosphonium cation having 1 to 32 carbon atoms, a tetraalkylammonium cation having 1 to 32 carbon atoms, and a trialkylsulfonium cation having 1 to 32 carbon atoms. Examples of the anion for the ionic liquid include halides ($F^-$, $Cl^-$, $Br^-$, $I^-$), formate, acetate, nitrate, phosphate, sulfate, sulfonate, tetrafluoroborate, hexafluorophosphate, triflate, bis(trifluoromethylsulfonyl)imide, tosylate, alkylsulfonate anion, alkylsulfate anion, carboxylate anion, and phthalate anion.

Specific examples of the ionic liquid as the anionic polymerization initiator include an imidazolium salt represented by the following General Formula (A), a pyrazolium salt represented by the following General Formula (B), a pyridinium salt represented by the following General Formula (C), a pyrimidinium salt or pyrazinium salt represented by the following General Formula (D), and an ammonium salt or phosphonium salt represented by the following General Formula (E).

[Chemical Formula 8]

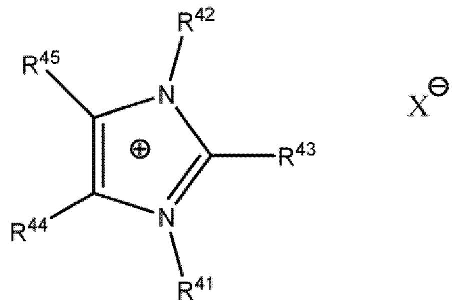

(A)

wherein in Formula (A), $R^{41}$ and $R^{42}$ each independently represent an alkyl group having 1 to 12 carbon atoms; $R^{43}$, $R^{44}$, and $R^{45}$ each independently represent a hydrogen atom or an alkyl group having 1 to 12 carbon atoms; and X represents an anion.

[Chemical Formula 9]

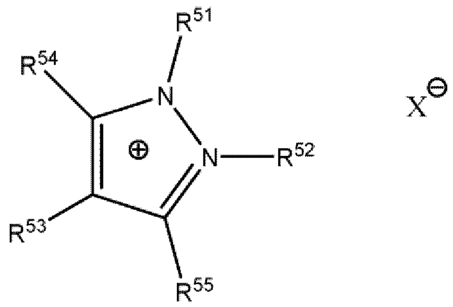

(B)

wherein in Formula (B), $R^{51}$ and $R^{52}$ each independently represent an alkyl group having 1 to 12 carbon atoms; $R^{53}$, $R^{54}$, and $R^{55}$ each independently represent a hydrogen atom or an alkyl group having 1 to 12 carbon atoms; and X represents an anion.

[Chemical Formula 10]

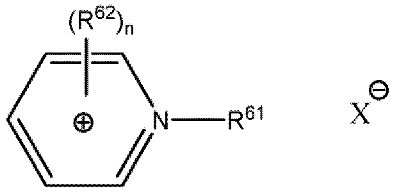

(C)

wherein in Formula (C), $R^{61}$ represents an alkyl group having 1 to 12 carbon atoms; $R^{62}$'S each independently represent a hydrogen atom or an alkyl group having 1 to 12 carbon atoms; n represents an integer from 0 to 5; and X represents an anion.

[Chemical Formula 11]

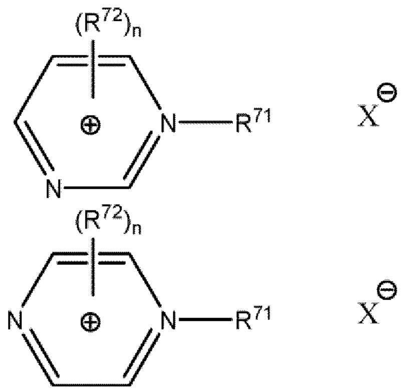

(D)

wherein in Formula (D), $R^{71}$ represents an alkyl group having 1 to 12 carbon atoms; $R^{72}$'S each independently represent a hydrogen atom or an alkyl group having 1 to 12 carbon atoms; n represents an integer from 0 to 4; and X represents an anion.

[Chemical Formula 12]

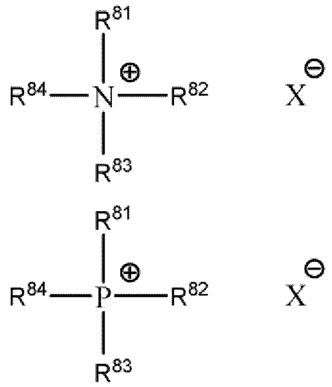

(E)

wherein in Formula (E), $R^{81}$, $R^{82}$, $R^{83}$, and $R^{84}$ each independently represent an alkyl group having 1 to 12 carbon atoms; and X represents an anion.

In the above-described Formulae (A) to (E), examples of the anion include halides ($F^-$, $Cl^-$, $Br^-$, $I^-$), formate, acetate, nitrate, phosphate, sulfate, sulfonate, tetrafluoroborate, hexafluorophosphate, triflate, bis(trifluoromethylsulfonyl)imide, tosylate, alkylsulfonate anion, alkylsulfate anion, carboxylate anion, and phthalate anion.

The cement-based molded body of the present disclosure is a product that is molded by performing casting of a cement-based composition. Examples of the cement-based molded body include molded bodies of mortar or concrete. Incidentally, the cement-based molded body may contain core materials such as a reinforcing bar and a steel frame.

The cement-based composition is not particularly limited; however, preferably the cement-based composition includes aggregate and water. Examples of the aggregate include fine aggregate and coarse aggregate. Incidentally, a concrete composition that includes fine aggregate and water but does not include coarse aggregate may be referred to as mortar.

Examples of the cement include Portland cement such as ordinary, low heat, moderate heat, high early strength, ultrahigh early strength, and sulfate-resistant; blast furnace cement, silica cement, fly ash cement, eco cement, and silica fume cement.

Examples of the fine aggregate include river sand, mountain sand, sea sand, crushed sand, heavyweight aggregate, lightweight aggregate, slag aggregate, and recycled aggregate.

Examples of the coarse aggregate include river gravel, crushed stone, heavyweight aggregate, lightweight aggregate, slag aggregate, and recycled aggregate.

Examples of water include tap water shown in Annex 9 of JIS A 5308, water other than tap water (river water, lake water, well water, or the like), and recovered water.

Any appropriate additives may also be added to the cement-based composition. Examples include a hardening accelerator, a setting retarder, a rust preventive agent, a waterproofing agent, an antiseptic agent, and a powder. Examples of the powder include silica fume, fly ash, limestone fine powder, blast furnace slag fine powder, expanding material, and other mineral fine powders.

<Method for producing cement-based structure with coating film>

The method for producing a cement-based structure with a coating film of the present disclosure includes a step of coating at least a portion of the surface of a cement-based molded body with the above-described curing agent and curing the cement-based molded body. As described above, since the curing agent of the present disclosure can effectively suppress dissipation of moisture from the surface of a cement-based molded body, a cement-based structure with a coating film, which has fewer defects such as cracks, is likely to be obtained.

The method for obtaining a cement-based molded body is not particularly limited, and a cement-based molded body can be obtained by performing casting by a known method. For example, a method of pouring a cement composition into a formwork for concrete casting, and hardening the cement-based composition, may be mentioned. In the case of obtaining reinforced concrete, steel-framed concrete, or the like, casting may be performed after a core material such as a reinforcing bar is disposed at a predetermined position inside a formwork.

After formwork removal, the cement-based molded body thus obtained is subjected to curing. The cement-based molded body may be subjected to initial curing such as underwater curing before curing. It is preferable to apply the curing agent over the entire surface of the cement-based molded body; however, dissipation of moisture may also be prevented by applying the curing agent only on at least a portion of the surface, while covering the unapplied surface

with a curing sheet or the like. Incidentally, the surface of the cement-based molded body may be roughly leveled and compacted before curing, and the surface may be leveled by performing shaving with a ruler or the like. Furthermore, curing may also be performed without removing the cement-based molded body from the formwork.

The coating amount of the curing agent is not particularly limited; however, the coating amount is preferably 50 to 500 g/$m^2$, more preferably 50 to 300 g/$m^2$, and even more preferably 50 to 200 g/$m^2$.

Shrinkage of a cement-based molded body during curing can be reduced by coating at least a portion of the surface of the cement-based molded body with the curing agent of the present disclosure. The shrinkage reduction rate of the cement-based molded body is preferably 23% or more, and preferably 25% or more, at the time point where the material age reaches 6 weeks (42 days have passed). Furthermore, drying of a cement-based molded body during curing can be suppressed by coating at least a portion of the surface of the cement-based molded body with the curing agent of the present disclosure. Suppression of drying can be evaluated by the successive change in mass (mass change rate) of the cement-based molded body during curing. The mass change rate is preferably 2.0% or less at the time point where the material age reaches 6 weeks (42 days have passed). Incidentally, the shrinkage reduction rate and the mass change rate are measured by the methods described in Examples.

As described above, a cement-based structure with a coating film is obtained by applying the curing agent on the surface of a cement-based molded body and then curing the cement-based molded body. The cement-based structure with a coating film of the present disclosure includes a cement-based structure and a coating film that covers at least a portion of the surface of the cement-based structure. The coating film includes a hardened product of the above-described curing agent. Meanwhile, the cement-based structure is a cement-based molded body after curing. The cement-based structure with a coating film of the present disclosure can be used as a construction material or the like for a building structure or the like.

Furthermore, after a coating film is formed by applying the curing agent of the present disclosure on a cement-based molded body, a coating film is formed as the coating film hardens. Since this coating film contains a polymer obtained by polymerizing a diester compound of Formula (I) (that is, a polymer containing a structural unit derived from a diester compound of Formula (I)), the strength and durability of the coating film tends to be higher, and the coating film can also function as a protective film at the surface of the cement-based molded body in the middle of curing and the produced cement-based structure.

For example, the function of the protective film may also be suppressing penetration of a deterioration factor into a cement-based molded body or a cement-based structure. That is, the curing agent of the present disclosure can also be used as a surface protective agent for suppressing penetration of a deterioration factor into a cement-based molded body or a cement-based structure. A deterioration factor is, for example, a substance that causes neutralization, salt damage, frost damage, chemical erosion, and alkali aggregate reaction of a cement-based molded body or a cement-based structure, and specific examples include carbon dioxide, chloride ion, an acid, an alkali, a sulfate, and water. Meanwhile, in a case where it is intended to suppress penetration of a deterioration factor, the curing agent of the present disclosure may be applied on a cement-based structure as a surface protective agent. Furthermore, those mentioned above as examples of the curing agent of the present disclosure can all be used also as surface protective agents. The coating amount of the surface protective agent is not particularly limited; however, the coating amount is preferably 50 to 500 g/$m^2$, more preferably 50 to 300 g/$m^2$, and even more preferably 50 to 200 g/$m^2$.

On the other hand, since the conventional curing agents described in Patent Literature 1 and the like are such that a coating film formed of oily components only is formed on the surface of a cement-based molded body by applying a wax, an emulsion, or the like, the strength and durability of the coating film thus formed are insufficient, and the coating film hardly functions as a protective film.

Incidentally, the diester compound of Formula (I) tends to spontaneously initiate a polymerization reaction after application of the curing agent, as a result of the action of a $Si-O^-$ group or the like at the surface of a cement-based molded body. In a case where an anionic polymerization reaction occurs due to the action of a $Si-O^-$group, a polymer containing a structural unit derived from a diester compound of Formula (I) acquires a structure of forming a covalent bond with a Si-O- group at the surface of the cement-based molded body. In other words, since covalent bonds are formed between the coating film and the cement-based molded body, a coating film having superior close adhesiveness and durability tends to be obtained.

After the curing agent is applied on a cement molded body to form a coating film, heating may be performed in order to accelerate hardening of the coating film. The heating temperature is preferably 100°C or lower and is preferably higher than room temperature (25°C). When the heating temperature is 100°C or lower, there is a tendency that the hardening time can be shortened while side reactions and the like are suppressed.

## Examples

Hereinafter, the present invention will be described in more detail by way of Examples; however, the present invention is not intended to be limited to these Examples. Meanwhile, unless particularly stated otherwise, parts and

percent (%) as used in Examples are on a mass basis.

<Production of mortar specimen>

450 g of ordinary Portland cement (manufactured by TAIHEIYO CEMENT CORPORATION), 225 g of water, and 1350 g of standard sand for cement strength test (defined in JIS R5201-1997 Annex 2-5.1.3: Japan Cement Association) were subjected to kneading of mortar according to the method of JIS R5201-1997 using a Hobart type mortar mixer (manufactured by Hobart, product number: N-50).

According to JIS A1129, a mortar specimen (4 × 4 × 16 cm) was produced. Silicone grease was applied in advance on a formwork so as to stop water and to allow the formwork to be easily removed. Furthermore, gauge plugs were attached at the two ends of the specimen. A formwork into which the mortar obtained by kneading was poured was placed in a container, tightly sealed, and stored at 20°C, and the mortar was subjected to initial curing. After one day, the formwork was removed, the silicone grease adhering to the specimen was washed with water using a scrubbing brush, and subsequently the specimen was cured in still water (underwater curing) at 20°C for 6 days. Thereby, a mortar specimen was obtained.

(Example 1 and Comparative Example 1)

A specimen on which a coating film was formed using the curing agent shown in Table 1 over the entire surface of the above-described mortar specimen was prepared as Example 1. The coating film was formed by wiping out water on the surface of the mortar specimen cured in water for 6 days with a paper towel, subsequently immediately dropping the curing agent thereon, spreading the dropped curing agent uniformly with a polyethylene terephthalate film, and leaving the specimen to stand. Furthermore, in Comparative Example 1, the curing agent was not used for the above-described mortar specimen, and a coating film was not formed thereon. Meanwhile, the abbreviations used in Table 1 are as follows.

DHMM: Di-n-hexyl methylenemalonate (Tg = -45°C)
DCHMM: Dicyclohexyl methylenemalonate (Tg = 140°C)

[Table 1]

| | Curing agent | Coating amount | |
|---|---|---|---|
| | | Overall (g) | Per unit area (g/m$^2$) |
| Example 1 | DHMM : DCHMM = 1 : 1 (mass ratio) | 2.6 | 96 |
| Comparative Example 1 | - | 0 | 0 |

<Measurement of shrinkage reduction rate and mass change rate>

According to JIS A1129, water on the surface of each mortar specimen that had been cured in still water for 6 days was wiped with a paper towel, subsequently the length was measured immediately using a dial gauge (manufactured by nishinihonshikenki), and this length was designated as the length of each mortar specimen at the time point of material age 0. With regard to the mortar specimen of Example 1, after the length was measured, the curing agent was immediately applied thereon as described above. Subsequently, the mortar specimen was stored in a constant-temperature constant-humidity chamber set at a temperature of 20°C and a humidity of 60%, and the mortar specimen was measured in a timely manner according to the various material ages shown in Table 2. For each mortar specimen, the difference between the length at each material age and the length at the time point of material age 0 was designated as the amount of shrinkage at each material age. Then, as shown by the following formula, the reduction proportion of the shrinkage strain with respect to a mortar specimen in a case where a curing agent was not used (that is, mortar specimen of Comparative Example 1) was designated as shrinkage reduction rate. The results are shown in Table 2.

Shrinkage reduction rate

= {(Amount of shrinkage of mortar specimen in case where curing agent was not used - amount of shrinkage of mortar specimen in case of using curing agent)/(amount of shrinkage of mortar specimen in case where curing agent was not used)} × 100 (%)

At the same time as the shrinkage reduction rate, the mass of the specimen was measured at various material ages, and the mass reduction rate was calculated by the following formula. As this mass reduction rate is larger, it is implied that evaporation of moisture from the specimen occurs to a larger extent. The results are shown in Table 3.

$$\text{Mass reduction rate (\%)} = \{(W_0 - W_X)/W_0\} \times 100$$

$W_0$: Mass of specimen on day 0 of material age (g)
$W_x$: Mass of specimen on day x of material age (g)

[Table 2]

| | Shrinkage reduction rate (%) | | | |
|---|---|---|---|---|
| | Material age 1 week | Material age 2 weeks | Material age 4 weeks | Material age 6 weeks |
| Example 1 | 68.1 | 46.1 | 36.8 | 26.3 |
| Comparative Example 1 | 0 | 0 | 0 | 0 |

[Table 3]

| | Mass change rate (%) | | | |
|---|---|---|---|---|
| | Material age 1 week | Material age 2 weeks | Material age 4 weeks | Material age 6 weeks |
| Example 1 | 0.5% | 1.0% | 1.5% | 1.8% |
| Comparative Example 1 | 1.9% | 2.6% | 2.8% | 3.0% |

<Test for hardenability at mortar surface>

A curing agent was dropped on the top surface of a mortar specimen, and a polyethylene terephthalate (PET) film (E7002 manufactured by TOYOBO CO., LTD.) coated with a release agent on one surface was placed on the dropped curing agent and left to stand. The ease of peeling of the PET film and the state of the coating film when touched with a finger were checked, and the hardenability of the coating film and the time required for the coating film to harden (curing time) were measured. Whether the coating film had hardened was judged by regarding the state in which no deposits originating from the curing agent were observed on the film when the PET film was peeled off, and the state in which no deposits attached when the coating film was touched with a finger, as hardening. The results are shown in Table 4. Meanwhile, with regard to the hardenability of the coating film and the hardening time, measurement was made for both a case where the surface of the mortar specimen was dry ("Dry" in Table 4) and a case where the surface of the mortar specimen was wet ("Wet" in Table 4). The evaluation criteria for hardenability are as follows. Furthermore, the terms "Dry" and "Wet" refer to a specimen that has been subjected to the following operations.

A: Hardened within 5 hours after application.
B: Hardened in about one day after application.
C: Only partially hardened even after one day passed after application.
Dry: A specimen that had been left to stand for about one day in an open state after curing in water. The surface of the specimen was in a dry state.
Wet: A specimen that had been left to stand for about one day in a sealed state after curing in water. The surface

of the specimen was in a damp state; however, the surface was not so damp that water adhered to the hand when the specimen was touched by hand.

Meanwhile, the ratio of the various components for the curing agents of Reference Examples 3 and 4 is a mass ratio. Furthermore, BD-PES represents a polyfunctional methylenemalonic acid ester compound obtained by transesterification between 1,4-butanediol and diethyl methylenemalonate according to Example 4 of International Publication WO 2018/031101.

[Table 4]

| | Curing agent | Surface state | Hardenability | Hardening time |
|---|---|---|---|---|
| Reference Example 1 | DHMM | Dry | A | 1.5 hours |
| | | Wet | A | 1 hour |
| Reference Example 2 | DCHMM | Dry | B | 1 day |
| | | Wet | B | 1 day |
| Reference Example 3 | DHMM : BD-PES = 1 : 1 | Dry | A | 4 hours |
| | | Wet | A | 2 hours |
| Reference Example 4 | DHMM : DCHMM = 1 : 1 | Dry | A | 2 hours |
| | | Wet | A | 1.5 hours |
| Reference Comparative Example | BD-PES | Dry | C | - |
| | | Wet | C | - |

(Examples 2 to 7 and Comparative Examples 2 to 4)

As shown in Table 5, measurement of the shrinkage reduction rate and the mass change rate was carried out in the same manner as in Example 1, except that the composition or coating amount of the curing agent was changed. Meanwhile, the term "CRATECURE" in Table 5 is the trade name for a fat and oil surfactant blend manufactured by DKS Co. Ltd. Furthermore, the composition (blending ratio) of the curing agents in Table 5 is on the basis of mass ratio. The results are shown in Table 6 and Table 7, respectively.

[Table 5]

| | Composition (blending ratio) | Coating amount | |
|---|---|---|---|
| | | Total amount (g) | Per unit area ($g/m^2$) |
| Example 2 | DHMM | 2.6 | 100 |
| Example 3 | DHMM | 3.8 | 150 |
| Example 4 | DCHMM | 2.6 | 100 |
| Example 5 | DCHMM | 3.8 | 150 |
| Example 6 | DHMM : DCHMM = 1 : 1 | 3.8 | 150 |
| Example 7 | DHMM : DCHMM : BD-PES = 45 : 45 : 10 | 3.8 | 150 |
| Comparative Example 2 | CRATECURE | 2.6 | 100 |
| Comparative Example 3 | CRATECURE | 3.8 | 150 |
| Comparative Example 4 | CRATECURE | 5.1 | 200 |

[Table 6]

| | Shrinkage reduction rate | | | |
|---|---|---|---|---|
| | Material age 1 week | Material age 2 weeks | Material age 4 weeks | Material age 6 weeks |
| Example 2 | 49% | 33% | 25% | 22% |
| Example 3 | 60% | 45% | 31% | 28% |
| Example 4 | 66% | 51% | 33% | 27% |
| Example 5 | 62% | 43% | 35% | 29% |
| Example 6 | 68% | 49% | 33% | 31% |
| Example 7 | 54% | 40% | 26% | 22% |
| Comparative Example 2 | 14% | 7% | 4% | 3% |
| Comparative Example 3 | 43% | 22% | 18% | 13% |
| Comparative Example 4 | 41% | 25% | 19% | 15% |

[Table 7]

| | Mass change rate | | | |
|---|---|---|---|---|
| | Material age 1 week | Material age 2 weeks | Material age 4 weeks | Material age 6 weeks |
| Example 2 | 1.0% | 1.5% | 2.0% | 2.3% |
| Example 3 | 0.8% | 1.2% | 1.7% | 2.0% |
| Example 4 | 0.7% | 1.2% | 1.7% | 2.0% |
| Example 5 | 0.7% | 1.2% | 1.7% | 2.0% |
| Example 6 | 0.7% | 1.1% | 1.6% | 1.9% |
| Example 7 | 0.7% | 1.2% | 1.7% | 2.1% |
| Comparative Example 2 | 1.9% | 2.4% | 2.8% | 3.0% |
| Comparative Example 3 | 1.7% | 2.2% | 2.7% | 2.9% |
| Comparative Example 4 | 1.6% | 2.1% | 2.5% | 2.8% |

<Measurement of compressive strength>

587 g of ordinary Portland cement (manufactured by TAIHEIYO CEMENT CORPORATION), 264 g of water, and 1350 g of standard sand for cement strength test (defined in JIS R5201-1997 Annex 2-5.1.3: Japan Cement Association) were subjected to kneading of mortar according to the method of JIS R5201-1997 using a Hobart type mortar mixer (manufactured by Hobart, product number: N-50). The mortar thus prepared was charged to fill up to one-third of the formwork capacity of a cylindrical formwork (diameter 5 cm, height 10 cm) placed on a horizontal table, the mortar was poked twenty times using a tamping rod, and then vibration was applied to the formwork container to extract coarse air bubbles. The formwork was further packed with the mortar full to the brim, the mortar was poked twenty times using a tamping rod, and then vibration was applied to the formwork container. In order to prevent drying, the top surface was covered with a PET film, curing was performed for 24 hours in an environment at room temperature of 20°C and a humidity of 60%, and then curing was performed by the following various methods.

Example 8: The formwork container was removed, subsequently the curing agent shown in Table 8 was applied on the specimen surface (coating amount 100 g/$m^2$), and the specimen was cured for 27 days in an environment at room temperature of 20°C and a humidity of 60%.

Comparative Example 5: Curing was performed in the same manner as in Example 8, except that the curing agent was changed to CRATECURE.

Comparative Example 6 (curing in air): The formwork container was removed, and then the specimen was cured for 27 days in an environment at room temperature of 20°C and a humidity of 60%.

Using the specimens produced by this method, the compressive strength was measured according to a compressive strength measuring method for concrete tests (JIS A1108:2006). The compressive strength was measured for three specimens for each of the various curing methods, and the average value of the compressive strength was calculated. The results are shown in Table 8. Meanwhile, the composition of the curing agent in Table 8 is on the basis of a mass ratio.

[Table 8]

| | Curing agent | Coating amount (g/m$^2$) | Compressive strength (N/mm$^2$) | | | |
|---|---|---|---|---|---|---|
| | | | 1 | 2 | 3 | Average value |
| Example 8 | DHMM : DCHMM = 1:1 | 100 | 50.2 | 57.6 | 55.5 | 54.4 |
| Comparative Example 5 | CRATECURE | 100 | 48 | 47.8 | 46.1 | 47.3 |
| Comparative Example 6 | - (Curing in air) | - | 32.5 | 32.3 | 31.4 | 32.1 |

<Production of mortar specimen>

A mortar specimen was produced by a method according to JSCE-K571-2013. The mortar was prepared using 18.00 kg of ordinary Portland cement (manufactured by TAIHEIYO CEMENT CORPORATION), 9.00 kg of water, and 54.00 kg of standard sand for cement strength test (defined in JIS R5201-1997 Annex 2-5.1.3: Japan Cement Association) by kneading the materials for 90 seconds with a pan type forced kneading mixer (50 L).
The mortar was cured as a prismatic body having a size of 10 × 10 × 40 cm by a method according to JSCE-K571-2013 using a steel form, subsequently cutting was performed, and polishing and sealing of the surface (sealing of four surfaces excluding the test surface with an epoxy resin) were carried out.

(Examples 9 and 10 and Comparative Example 7)

As Examples 9 and 10, specimens in which a coating film was formed on two surfaces facing each other of the above-described specimens using a surface protective agent shown in Table 9, were prepared. The coating film was formed by wiping water on the surface of a mortar specimen that had been cured for 6 days in water with a paper towel, subsequently immediately dropping a surface protective agent thereon, uniformly spreading the dropped surface protective agent, and leaving the specimen to stand. The coating amount of the surface protective agent was 100 g/m$^2$ in all cases. Furthermore, in Comparative Example 7, a surface protective agent was not used for the above-described mortar specimen, and no coating film was formed.

<Accelerated neutralization test>

For each of the specimens obtained in Examples 9 and 10 and Comparative Example 7, an accelerated neutralization test was performed by a method according to JSCE-K571. The test period was set to 56 days, and the neutralization depth was measured by spraying a phenolphthalein solution on a cleaved surface of the specimen after 56 days and measuring the coloration area of the solution. The measurement results are shown in Table 9.

<Chloride ion penetration test>

For each of the specimens obtained in Examples 9 and 10 and Comparative Example 7, a chloride ion penetration test was performed by a method according to JSCE-K572. A specimen was immersed in a 10% NaCl solution, and after 63 days, the penetration depth of chloride ions was measured. The measurement results are shown in Table 9.

[Table 9]

| | Surface protective agent | Coating amount (g/m$^2$) | Neutralizatio n depth nm | Chloride ion penetration depth (nm) |
|---|---|---|---|---|
| Example 9 | DCHMM/DHMM = 1/1 (mass ratio) | 100 | 1.0 | 4.5 |
| Example 10 | DCHMM/DHMM/B D-PES = 45/45/10 (mass ratio) | 100 | 0.5 | 1.5 |

(continued)

| | Surface protective agent | Coating amount (g/$m^2$) | Neutralizatio n depth nm | Chloride ion penetration depth (nm) |
|---|---|---|---|---|
| Comparative Example 7 | - | - | 5.4 | 12.5 |

## Claims

1. A curing agent comprising a diester compound represented by the following Formula (I):

[Chemical Formula 1]

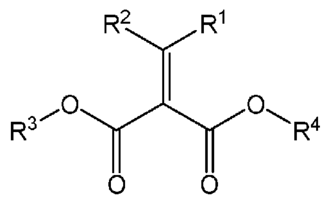

• • • ( I )

wherein in Formula (I), $R^1$ and $R^2$ each independently represent a hydrogen atom or a monovalent hydrocarbon group having 1 to 15 carbon atoms, or $R^1$ and $R^2$ are bonded together to form a divalent hydrocarbon group having 3 to 15 carbon atoms; and $R^3$ and $R^4$ each independently represent a monovalent organic group having 1 to 30 carbon atoms, or $R^3$ and $R^4$ are bonded together to form a divalent organic group having 3 to 30 carbon atoms.

2. The curing agent according to claim 1,

   wherein the diester compound includes a first diester compound, and
   the first diester compound satisfies at least one of the following conditions (1) and (2):

   (1) the glass transition temperature of a homopolymer is lower than 30°C; and
   (2) $R^3$ and $R^4$ each independently represent a monovalent linear or branched alkyl group having 3 to 30 carbon atoms.

3. The curing agent according to claim 2, wherein the diester compound further includes a second diester compound, a homopolymer of the second diester compound having a glass transition temperature of 60°C or higher.

4. The curing agent according to claim 3, wherein the glass transition temperature of a homopolymer of the second diester compound is 100°C or higher.

5. The curing agent according to any one of claims 1 to 4, further comprising a polyfunctional methylenemalonic acid ester compound containing two or more of a structural unit represented by the following Formula (II) in the molecule and having the two or more structural units linked by a residue of a polyhydric alcohol:

[Chemical Formula 2]

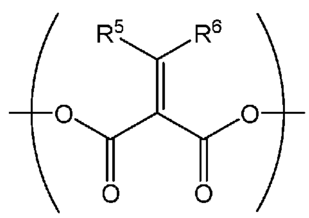

• • • ( I I )

wherein in Formula (II), $R^5$ and $R^6$ each independently represent a hydrogen atom or a monovalent hydrocarbon group having 1 to 15 carbon atoms.

6. A method for producing a cement-based structure with a coating film, the method comprising a step of coating at least a portion of the surface of a cement-based molded body with the curing agent according to any one of claims 1 to 5 and curing the cement-based molded body.

7. A method for reducing shrinkage of a cement-based molded body during curing by coating at least a portion of the surface of a cement-based molded body with the curing agent according to any one of claims 1 to 5.

8. A method for suppressing drying of a cement-based molded body during curing by coating at least a portion of the surface of a cement-based molded body with the curing agent according to any one of claims 1 to 5.

9. A method for suppressing penetration of a deterioration factor into a cement-based structure by coating at least a portion of the surface of the cement-based structure with a surface protective agent containing a diester compound represented by the following Formula (I):

[Chemical Formula 3]

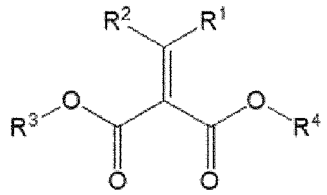

・・・(I)

wherein in Formula (I), $R^1$ and $R^2$ each independently represent a hydrogen atom or a monovalent hydrocarbon group having 1 to 15 carbon atoms, or $R^1$ and $R^2$ are bonded together to form a divalent hydrocarbon group having 3 to 15 carbon atoms; and $R^3$ and $R^4$ each independently represent a monovalent organic group having 1 to 30 carbon atoms, or $R^3$ and $R^4$ are bonded together to form a divalent organic group having 3 to 30 carbon atoms.

**INTERNATIONAL SEARCH REPORT**

International application No.
PCT/JP2020/003038

A. CLASSIFICATION OF SUBJECT MATTER
Int.Cl. C04B40/04(2006.01)i, B28B11/24(2006.01)i, E04G21/02(2006.01)i
FI: C04B40/04, B28B11/24, E04G21/02104

According to International Patent Classification (IPC) or to both national classification and IPC

B. FIELDS SEARCHED

Minimum documentation searched (classification system followed by classification symbols)
Int.Cl. C04B40/00-40/06, C04B41/00-41/72, B28B11/00-11/24, E04G21/02-21/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2017/184986 A1 (ZEPHYROS, INC.) 26.10.2017 (2017-10-26), entire text | 1-9 |
| A | JP 56-69258 A (SHIN ETSU CHEMICAL CO., LTD.) 10.06.1981 (1981-06-10), entire text | 1-9 |
| P, A | WO 2019/129572 A1 (BASF SE) 04.07.2019 (2019-07-04), entire text | 1-9 |
| P, A | WO 2019/129539 A1 (BASF SE) 04.07.2019 (2019-07-04), entire text | 1-9 |
| P, A | WO 2019/129537 A1 (BASF SE) 04.07.2019 (2019-07-04), entire text | 1-9 |

☐ Further documents are listed in the continuation of Box C.

☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18.03.2020 | Date of mailing of the international search report<br>31.03.2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2020/003038

| | | |
|---|---|---|
| WO 2017/184986 A1 | 26.10.2017 | US 2019/0169396 A1<br>EP 3445830 A1<br>CN 109476955 A |
| JP 56-69258 A | 10.06.1981 | (Family: none) |
| WO 2019/129572 A1 | 04.07.2019 | (Family: none) |
| WO 2019/129539 A1 | 04.07.2019 | (Family: none) |
| WO 2019/129537 A1 | 04.07.2019 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010018490 A
- WO 2018031101 A